# EUROPEAN PATENT APPLICATION

(11) **EP 4 089 622 A1**
(43) Date of publication of application: **16.11.2022**
(21) Application number: 20911547.6
(22) Date of filing: 30.12.2020
(51) Int. Cl.: G06Q 50/22, G06Q 50/10, G06Q 30/08, G06Q 50/00, G06Q 50/30, C12Q 1/6827

(54) **PET GENOME-BASED MATCHMAKING SOCIAL NETWORK SYSTEM AND METHOD FOR PROVIDING PET GENOME-BASED MATCHMAKING INFORMATION**

(30) Priority: 09.01.2020 KR 20200003218
(71) Applicant: Clinomics Inc., Ulsan 44919 (KR)
(72) Inventor: KIM, Byoung Chul, Ulju-gun Ulsan 44921 (KR); CHOI, Su Bin, Ulsan 44025 (KR); KIM, Chang Jae, Ulju-gun Ulsan 44920 (KR); BHAK, Jong Hwa, Ulju-gun Ulsan 44936 (KR); BHAK, Young June, Ulsan 44686 (KR); AN, Ji Hye, Ulsan 44476 (KR); UM, Hyo Jin, Ulsan 44780 (KR); CHOI, Ju Young, Ulju-gun Ulsan 44922 (KR)
(74) Representative: Frenkel, Matthias Alexander
(86) International application number: PCT/KR2020/019385
(87) International publication number: WO 2021/141316

(57) **Abstract**

The present invention relates to a pet genome-based matchmaking social network system and a method for providing pet genome-based matchmaking information, the system comprising: a gene analysis device unit which provides pet gene analysis information; a bioinformatics information analysis device unit which provides information that may appear during breeding through bioinformatics analysis based on the gene analysis information; a basic information input unit which receives various information about pets from pet owners; a database unit which stores collected and analyzed information; and a pet matching recommendation unit which recommends a pet with the highest matching degree according to an application for matching.

## Description

### TECHNICAL FIELD

Embodiments of the present invention relate to a genome-based matchmaking social network system for pets and a method for providing genome-based matchmaking information for pets.

### BACKGROUND ART

In general, it is not easy to find, online, a counterpart pet meeting desired conditions for pet breeding.

For example, it is difficult to find a target meeting various detailed conditions for breeding a specific species of dog--for example, it should be the same species, as far a genetic distance as possible, small in size, a low chance of genetic disease, such as a knee joint disease, a specific color for children, and a dog with a good bowel movement.

Although a desired pet is found, it is impossible to check whether their children have a genetic problem.

### [PRIOR TECHNICAL DOCUMENTS]

Korean Patent Application Publication No. 10-2005-0110260 (published on November 23, 2005)
Korean Patent Application Publication No. 10-2004-0027593 (published on April 1, 2004)

### DETAILED DESCRIPTION OF THE INVENTION

### TECHNICAL PROBLEMS

Embodiments of the present invention provide a genome-based matchmaking information providing system for pets and genome-based matchmaking information providing method for pets, which may obtain genetically, externally, introvertedly, and physically specific breeding results based on genetic information, non-genetic information, epigenetic information about the pet and basic information about the pet owner and enables matching with a pet that has a low genetic risk and has a high chance of obtaining healthy offspring by breeding.

### MEANS TO ADDRSS THE PROBLEMS

According to an embodiment of the present invention, a pet genome-based matchmaking social network system comprises a gene analyzer that provides gene analysis information through gene analysis using a specimen collected from a pet; a bioinformatics information analyzer that provides at least one of genetic variation feature information, external feature information, and introverted feature information that appears upon breeding through bioinformatic analysis based on the gene analysis information; a basic information input unit that receives user basic information about an owner of the pet and genetic feature information and non-genetic feature information about the pet from the owner of the pet; a database unit that collects and stores information obtained and inputted through the gene analyzer, the bioinformatics information analyzer, and the basic information input unit; and a pet matching recommendation unit that receives a matching request from a matching applicant, receives at least one condition information among genetic condition information, non-genetic condition information, external condition information, and introverted condition information about a desired matching target to recommend a pet with a highest degree of matching with the condition information among the information stored in the database unit or obtains analysis information through the genetic analyzer and the biogenetics information analyzer using a specimen collected from the matching applicant's pet to recommend a pet with a highest degree of matching with the analysis information among the information stored in the database unit.

The pet genome-based matchmaking social network system further comprise an epigenetic analyzer that provides growth and physiological expression feature information about the pet through epigenetic analysis using the specimen collected from the pet, wherein the database unit additionally collects and stores the growth and physiological expression feature information.

The growth and physiological expression feature information may include information according to a result of a structural change in chromatin due to DNA methylation or histone protein modification.

The pet matching recommendation unit may analyze, predict, and additionally provide at least one of genetic disease risk information for each pet based on the information stored in the database unit and virtual external feature information, introverted feature information, health feature information, physical feature information, genetic disease risk information, and growth and physiological expression feature information for offspring resultant from breeding with a recommended pet.

The pet matching recommendation unit may calculate a breeding fitness between pets according to a heterogeneity of a major histocompatibility complex (MHC) gene and include and provide the breeding fitness in the virtual genetic disease risk information for the offspring resultant from breeding.

According to another embodiment of the present invention, a pet genome-based matchmaking information providing method may comprise a gene analysis step that provides gene analysis information through gene analysis using a specimen collected from a pet, by a gene analyzer; a bioinformatics information analysis step that provides at least one of genetic variation feature information, external feature information, and introverted feature information that appears upon breeding through bioinformatic analysis based on the gene analysis information, by a biogenetics information analyzer; a basic information input step that receives user basic information about an owner of the pet and genetic feature information and non-genetic feature information about the pet from the owner of the pet, by a basic information input unit; an information collection and storage step that collects and stores information obtained and inputted through the gene analyzer, the bioinformatics information analyzer, and the basic information input unit, by a database unit; and a pet matching recommendation step that, by a pet matching recommendation unit, receives a matching request from a matching applicant, receives at least one condition information among genetic condition information, non-genetic condition information, external condition information, and introverted condition information about a desired matching target to recommend a pet with a highest degree of matching with the condition information among the information stored in the database unit or obtains analysis information through the genetic analyzer and the biogenetics information analyzer using a specimen collected from the matching applicant's pet to recommend a pet with a highest degree of matching with the analysis information among the information stored in the database unit.

The pet genome-based matchmaking information providing method may further comprise an epigenetic analysis step that provides growth and physiological expression feature information about the pet through epigenetic analysis using the specimen collected from the pet, by an epigenetic analyzer, wherein the information collection and storage step additionally collects and stores the growth and physiological expression feature information.

The growth and physiological expression feature information may include information according to a result of a structural change in chromatin due to DNA methylation or histone protein modification.

The pet matching recommendation step may analyze, predict, and additionally provide at least one of genetic disease risk information for each pet based on the information stored in the database unit and virtual external feature information, introverted feature information, health feature information, physical feature information, genetic disease risk information, and growth and physiological expression feature information for offspring resultant from breeding with a recommended pet.

The pet matching recommendation step may calculate a breeding fitness between pets according to a heterogeneity of a major histocompatibility complex (MHC) gene and include and provide the breeding fitness in the virtual genetic disease risk information for the offspring resultant from breeding.

### EFFECTS OF THE INVENTION

According to the present invention, it is possible to provide a genome-based matchmaking information providing system for pets and genome-based matchmaking information providing method for pets, which may obtain genetically, externally, introvertedly, and physically specific breeding results based on genetic information, non-genetic information, epigenetic information about the pet and basic information about the pet owner and enables matching with a pet that has a low genetic risk and has a high chance of obtaining healthy offspring by breeding.

### BRIEF DESCRIPTION OF THE DRAWINGS

FIG. 1 is a schematic diagram illustrating an overall configuration of a pet genome-based matchmaking social network system according to an embodiment of the present invention.
FIG. 2 is a block diagram illustrating an overall configuration of a pet genome-based matchmaking social network system according to an embodiment of the present invention.
FIG. 3 is a view illustrating a phenotype visualization image of an offspring according to a virtual breeding matching result, according to an embodiment of the present invention.
FIGS. 4 and 5 are views illustrating a genetic result of breeding according to the genetic distance.
FIG. 6 is a view illustrating a matching result according to a genetic risk degree and a desired condition fitness according to an embodiment of the present invention.
FIG. 7 is a flowchart illustrating a pet genome-based matchmaking information providing method according to another embodiment of the present invention.

### MODE TO PRACTICE THE INVENTION

The terms used herein are briefly described, and the present invention is then described in detail.

For use in embodiments of the present invention, common terms widely used as possible have been chosen considering functions in the disclosure, but the terms may be varied depending on the intent of one of ordinary skill in the art or case laws or the advent of new technologies. In certain cases, some terms may be arbitrarily selected by the applicant, and in such case, their detailed definitions may be given in the relevant parts thereof. Accordingly, the terms used herein should be determined based on their meanings and the overall disclosure, rather than by the terms themselves.

When an element "includes" another element, the element may further include the other element, rather excluding the other element, unless particularly stated otherwise. Further, the terms "unit," "module," or "part" as used herein denote a unit processing at least one function or operation, and a unit, module, or part may be implemented in hardware, software, or a combination thereof.

Embodiments of the present invention are now described with reference to the accompanying drawings in such a detailed manner as to be easily practiced by one of ordinary skill in the art. However, the present invention may be implemented in other various forms and is not limited to the embodiments set forth herein. For clarity of the disclosure, irrelevant parts are removed from the drawings, and similar reference denotations are used to refer to similar elements throughout the specification.

As used herein, the terms "A or B," "at least one of A and/or B," or "one or more of A and/or B" may include all possible combinations of A and B. For example, "A or B," "at least one of A and B," "at least one of A or B" may indicate all of (1) including at least one A, (2) including at least one B, or (3) including at least one A and at least one B.

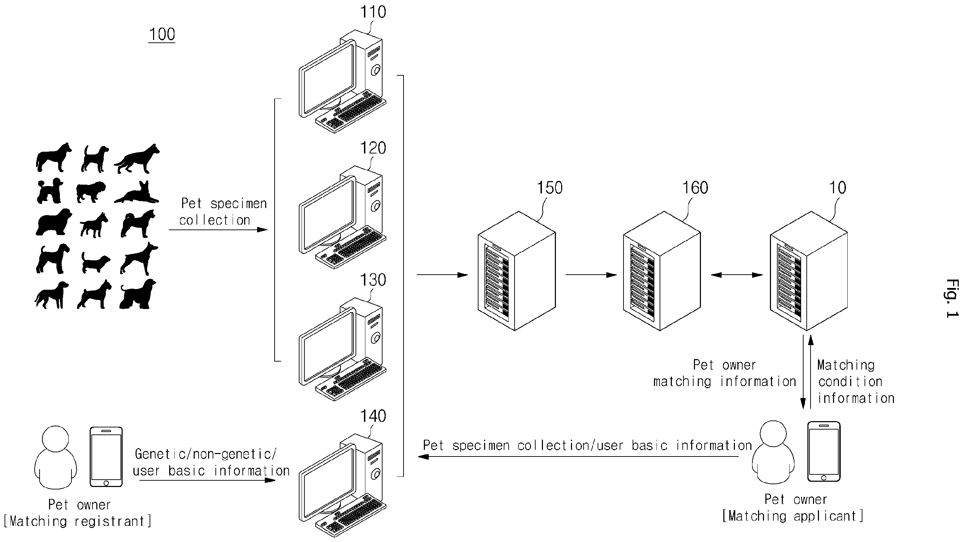

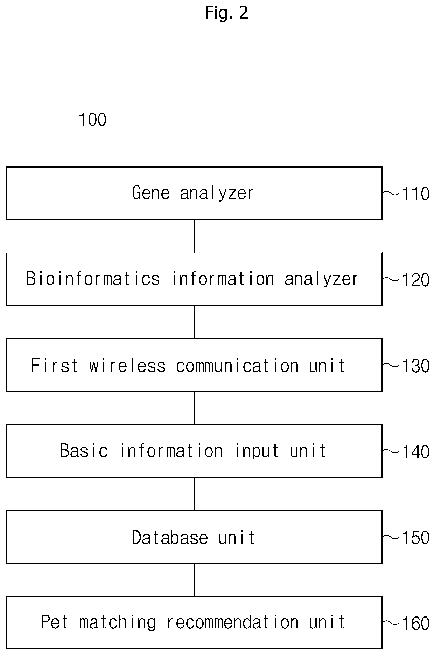

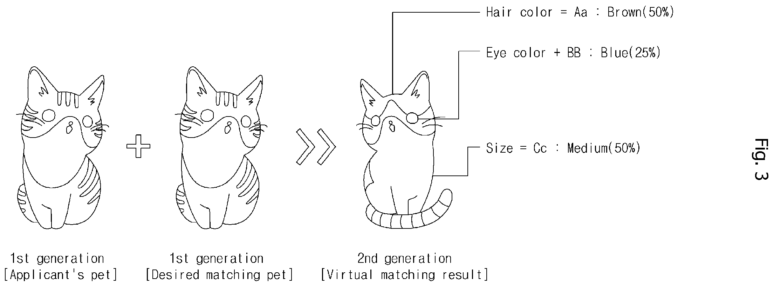

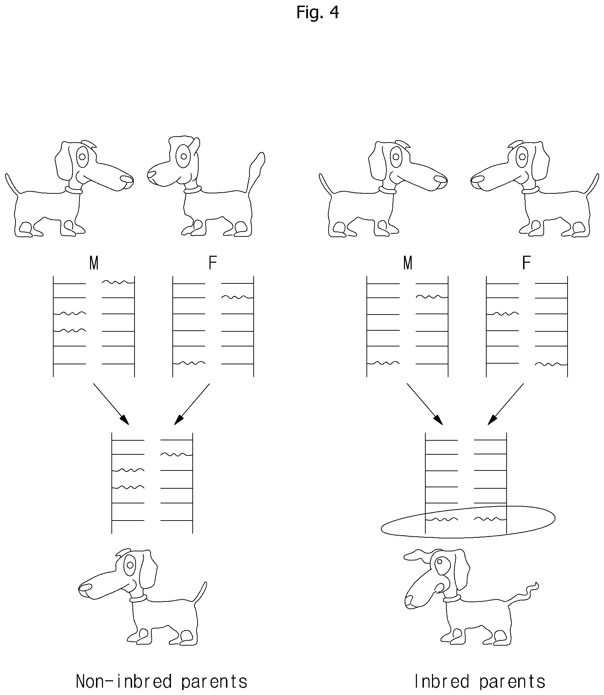

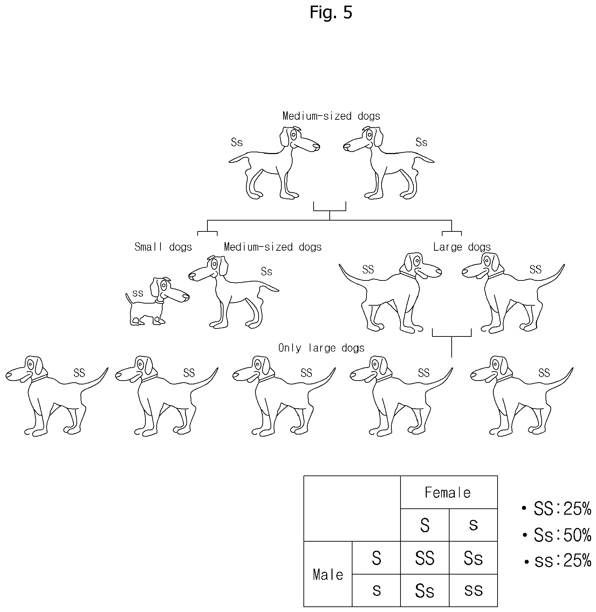

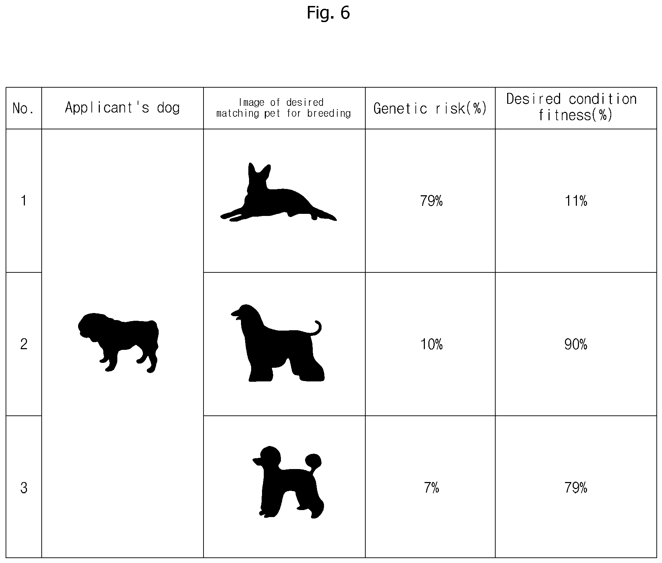

FIG. 1 is a schematic diagram illustrating an overall configuration of a pet genome-based matchmaking social network system according to an embodiment of the present invention. FIG. 2 is a block diagram illustrating an overall configuration of a pet genome-based matchmaking social network system according to an embodiment of the present invention. FIG. 3 is a view illustrating a phenotype visualization image of an offspring according to a virtual breeding matching result, according to an embodiment of the present invention. FIGS. 4 and 5 are views illustrating a genetic result of breeding according to the genetic distance. FIG. 6 is a view illustrating a matching result according to a genetic risk degree and a desired condition fitness according to an embodiment of the present invention.

Referring to FIGS. 1 and 2, a pet genome-based matchmaking social network system 100 according to an embodiment of the present invention may include a gene analyzer 110, a bioinformatics information analyzer 120, an epigenetic analyzer 130, a basic information input unit 140, a database unit 150, and a pet matching recommendation unit 160.

The gene analyzer 110 may provide gene analysis information through gene analysis using specimens collected from a pet. The types of specimens collected from the pet may include samples from which genetic information may be obtained, such as pet blood, saliva, defecation, and hair follicles (hair). The gene analyzer 110 may perform gene sequencing/genotyping and genetic information analysis on each pet using the samples, and as some specific methods, well-known gene analysis methods, such as WGS, Chip, BWA mapping, or GATK calling, may be carried out.

Further, the gene analyzer 110 may compare the genetic information about other pets stored in the database unit 150, which is described below, through a known technique, such as Identical By Descendent, thereby calculating the genetic distance or degree of kinship between the pets.

The bioinformatics information analyzer 120 may provide at least one of genetic variation feature information, external feature information, and introverted feature information that may appear during breeding through bioinformatics analysis based on the gene analysis information.

The epigenetic analyzer 130 may provide information about the growth and physiological expression feature of the pet through epigenetics analysis using the specimen collected from the pet. In the embodiment, growth and physiological expression feature information, that is, epigenetic information, may be information about a gene regulatory mechanism including information according to a result of a structural change in chromatin by DNA methylation or histone protein modification, as separate from pet DNA information.

Considering that the pet's disposition or physiological expression may be epigenetically more diversified (regulation of gene expression) by changes in the chromatin structure due to DNA methylation or modification of histone proteins although the base sequence of DNA is not changed because it is not genetically inherited from the parents, information about the results of structural changes in chromatin by DNA methylation or histone protein modification may be stored in the database unit 150 as described above. Further, all information about the pet may be recorded based on the fact that traits or characteristics obtained by the environment and training may be inherited.

For example, mice that were frequently licked by their parents when they were young will have a relaxed character and strong stress-resistance traits when they grow up. In other words, as the ERα gene regulatory mechanism variation changes, the GR gene increases, and stress is reduced, leading to different results from the beginning. Pups born from the parents that have been trained to bark less as compared with their species traits will develop the same less barking traits.

The basic information input unit 140 may receive, from the pet owner, genetic feature information and non-genetic feature information about the pet, and user basic information about the pet owner. The genetic feature information may include the type and breed of the pet. The non-genetic feature information may include photos of the pet, weight, presence of disease, physical characteristics, personality characteristics, the environment in which it was raised, training to learn, eating habits, sleeping habits, specific behaviors, and the like. The user basic information may include the name, identity, and residence of the pet owner.

The database unit 150 may collect and store the information obtained and input through the gene analyzer 110, the bioinformatics information analyzer 120, and the basic information input unit 130 and may additionally collect and store the growth and physiological expression feature information provided through the epigenetic analyzer 130.

The database unit 150 may continuously collect and add corresponding information by using theses published through a DB containing generally known genomic variation data for each pet type and related phenotypic information. The information stored in the database unit 150 may be compared with secured genetic variation information (bioinformatic information) about the pet, and pre-secured genetic variation information and potential phenotype may be added to the secured animal information list. Further, the database unit 150 may store and share the secured, added, and analyzed pet information in a pet genome matchmaker database unit 10 connected to a publicly available social network.

The pet matching recommendation unit 160 may receive a matching request from a matching applicant, receive at least one of genetic condition information, non-genetic condition information, external condition information, and introverted condition information about the desired matching target, and recommend a pet having the highest degree of matching with each condition information among the information stored in the database unit 150. In other words, it is possible to find and provide a pet that matches the conditions of the matching applicant in such a manner as to perform manual filtering according to the conditions or criteria desired by the matching applicant. As such, the pet matching recommendation unit 160 may score the matching fitness between the conditions desired by the matching applicant, that is, genetic information, non-genetic information, external condition information, and introverted condition information, such as the type, breed, age, presence of disease, physical characteristics, and personality characteristics about the pet desired for matching, and the genetic information, non-genetic information, external condition information, and introverted condition information about each pet stored in the database unit 150, thereby providing information about the fittest pets.

Further, the pet matching recommendation unit 160 may obtain analysis information through the genetic analyzer 110 and the bioinformatics information analyzer 120 using the specimens collected from the pet of the matching applicant and recommend the pet with the highest degree of matching with the analysis information among the information stored in the database unit 150 or an appropriate number of pets in order from the highest matching degree. In other words, the pet matching recommendation unit 160 may automatically filter and provide a list of a few highest pets with a high degree of fitness based on various analysis information and input information.

Further, as the basis for judging the matching fitness, it is possible to search, filter, and provide information about the pet suitable for breeding with the matching applicant's pet through the process of scoring the matching fitness for the above-described epigenetic information (traits or characteristics obtained by environment and training) and bioinformatics information (information, such as genetic variation, physical characteristics, and characters that may appear during breeding).

Meanwhile, the pet matching recommendation unit 160 may analyze, predict, and additionally provide at least one of genetic disease risk information about each pet based on the information stored in the database unit 150, and virtual appearance feature information, introverted feature information, health feature information (longevity potential), physical feature information (intelligence, body mass index, body hair thickness), and genetic disease risk information about the offspring from breeding with the recommended pet. In other words, it is possible to provide, in the form of a report, information about the potential genetic risk to the animal as well as the genetic risk to the offspring to be born by breeding with the recommended pet, through the genetic information about the matching applicant's pet.

Further, the pet matching recommendation unit 160 may provide a visualization service for predicting the shape of the offspring based on the genetic information about the mating target. As shown in FIG. 3, it is possible to predict, visualize, and provide external information about the offspring of the second generation that are born when two first-generation cats virtually breed. More specifically, it is possible to provide a visualization service (providing photos or images) for the phenotype of the second generation that may appear by breeding, based on the shape of the ears, the color of the hair, and the shape of the nose. Such visual information may be provided by predicting the health, character, and physical characteristics of the offspring to be born at the time of breeding.

Meanwhile, the pet matching recommendation unit 160 may also provide a visualization system for predicting the shape of the offspring based on the non-genetic information about the mating target. For example, the appearance of the offspring to be born by breeding between a Chihuahua and a Jindo may be provided in a non-genetic photosynthesis scheme. If pet owners register two photos, the visualization service may predict and show an intermediate (mixed) face through a face synthesis algorithm based on the registered photo information.

The pet matching recommendation unit 160 may calculate the degree of breeding fitness between pets according to heterogeneity of the major histocompatibility complex (MHC) gene and include and provide it in the virtual genetic disease risk information for the offspring to be born by breeding of the pets. Here, the major histocompatibility complex is a polymorphic gene, so it may have a very large number of alleles. Almost all humans as well as animals have different MHC alleles. There are two main types, type I and type II. Type I exists in almost all cells, and the T cell recognizes the MHC-1 molecule positioned on the surface of each cell to determine whether it is its own cell or not. If it turns out that it is not its own cell, the T cell destroys the cell. Because of this, when an organ transplant is received from another person, rejection occurs and the rejection must be suppressed for life through specific drugs.

As such, MHC genes perform the function of controlling the immune system to recognize and defend against foreign invaders, such as viruses or bacteria. It is possible to determine the risk of a genetic disease to the offspring by calculating the fitness of breeding according to the heterogeneity of the MHC gene and provide information thereabout. In other words, it is to predict the risk of genetic diseases of the offspring. It is possible to quantify the genetic risk inferred from the polymorphisms of genetic diseases and general diseases that occur in pets and humans. More specifically, it is possible to calculate and provide the predictive genetic risk used in human genetic testing and the heritable risk through mutation derived from a rare disease, separately and in combination.

Meanwhile, the pet matching recommendation unit 160 according to the present embodiment may minimize the genetic disease according to the degree of kinship and the genetic disease probability score (carrier test), while maintaining the optimal pure blood breed.

Further, the pet matching recommendation unit 160 may interwork with a system implemented in the form of an application or website that matches a pet breeding target online, so that the consumer (pet owner) may easily check the genome/gene analysis result through reports or various media.

Further, the pet matching recommendation unit 160 may prevent genetic disease by examining the genetic information and non-genetic information based on the desired conditions for the fitness of breeding with a specific pet when breeding with the specific pet is desired and provide information about the risk of genetic diseases of current pets and the risk of future offspring (carrier test) according to the genotype of the future mating partner and may be used in various fields, such as breeding grounds, pet shops, and veterinary hospitals.

The genome-based matchmaking social network system 100 for pets according to the present embodiment may provide owners with a function of securing and authenticating data for proof of pedigree relationship (authentication) of the pet that has been sold and provide a portal function through which the pet owner may easily manage information about his/her pet through the database unit 150 that is a pet genealogy DB. In other words, it is possible to manage the information even after the death of the pet.

Further, it is possible to judge the degree of kinship between pets through sample analysis. The range of the degree of difference in pet genetic variation may be previously defined for each species (e.g., distance between breeds of dogs, distance between breeds of cats), and the distance of animals falling in the range may be provided as the concept of degree of kinship of pets.

Meanwhile, the genome-based matchmaking social network system 100 for pets according to the present embodiment is able to genetically prove and manage the genealogy (or pedigree) of animals with the most certainty and, If genomic information is stored when a pet dies, provide information related to the pet so that the animal closest to the pet may be adopted in the future.

Further, in the genome-based matchmaking social network system 100 for pets, the genetic information may be stored in a storage medium, such as USB drive, to be easily identified online or on computer when the pet dies in the future.

Further, when expensive racehorses or stallions as well as pets are stolen or replaced, ownership may be proved through genetic testing and comparison with registered DB information. It may be a very reliable genetic test because it uses hundreds of thousands of markers (genotypes/biochips) that utilize a large amount of genetic information or sequences the entire genome of a pet.

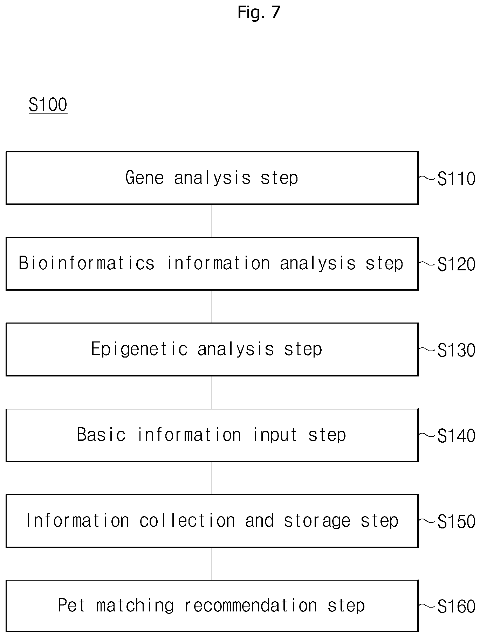

FIG. 7 is a flowchart illustrating a pet genome-based matchmaking information providing method according to another embodiment of the present invention.

Referring to FIG. 7, a genome-based matchmaking information providing method S100 for pets according to an embodiment of the present invention includes a gene analysis step S110, a bioinformatics information analysis step S120, an epigenetic analysis step S130, a basic information input step S140, an information collection and storage step S150, and a pet matching recommendation step S160.

The gene analysis step S110 may provide gene analysis information through gene analysis using specimens collected from a pet. The types of specimens collected from the pet may include samples from which genetic information may be obtained, such as pet blood, saliva, defecation, and hair follicles (hair). The gene analysis step S110 may perform gene sequencing/genotyping and genetic information analysis on each pet using the samples, and as some specific methods, well-known gene analysis methods, such as WGS, Chip, BWA mapping, or GATK calling, may be carried out.

Further, the gene analysis S110 may compare the genetic information about other pets stored in the information collection and storage step S150, which is described below, through a known technique, such as Identical By Descendent, thereby calculating the genetic distance or degree of kinship between the pets.

The bioinformatics information analysis step S120 may provide at least one of genetic variation feature information, external feature information, and introverted feature information that may appear during breeding through bioinformatics analysis based on the gene analysis information.

The epigenetic analysis step S130 may provide information about the growth and physiological expression feature of the pet through epigenetics analysis using the specimen collected from the pet. In the embodiment, growth and physiological expression feature information, that is, epigenetic information, may be information about a gene regulatory mechanism including information according to a result of a structural change in chromatin by DNA methylation or histone protein modification, as separate from pet DNA information.

Considering that the pet's disposition or physiological expression may be epigenetically more diversified (regulation of gene expression) by changes in the chromatin structure due to DNA methylation or modification of histone proteins although the base sequence of DNA is not changed because it is not genetically inherited from the parents, information about the results of structural changes in chromatin by DNA methylation or histone protein modification may be stored in the information collection and storage step S150 as described above. Further, all information about the pet may be recorded based on the fact that traits or characteristics obtained by the environment and training may be inherited.

For example, mice that were frequently licked by their parents when they were young will have a relaxed character and strong stress-resistance traits when they grow up. In other words, as the ERα gene regulatory mechanism variation changes, the GR gene increases, and stress is reduced, leading to different results from the beginning. Pups born from the parents that have been trained to bark less as compared with their species traits will develop the same less barking traits.

The basic information input step S140 may receive, from the pet owner, genetic feature information and non-genetic feature information about the pet, and user basic information about the pet owner. The genetic feature information may include the type and breed of the pet. The non-genetic feature information may include photos of the pet, weight, presence of disease, physical characteristics, personality characteristics, the environment in which it was raised, training to learn, eating habits, sleeping habits, specific behaviors, and the like. The user basic information may include the name, identity, and residence of the pet owner.

The information collection and storage step S150 may collect and store the information obtained and input through the gene analysis step S110, the bioinformatics information analysis step S120, and the basic information input step 130 and may additionally collect and store the growth and physiological expression feature information provided through the epigenetic analysis step S130.

The information collection and storage step S150 may continuously collect and add corresponding information by using theses published through a DB containing generally known genomic variation data for each pet type and related phenotypic information. The information stored in the information collection and storage step S150 may be compared with secured genetic variation information (bioinformatic information) about the pet, and pre-secured genetic variation information and potential phenotype may be added to the secured animal information list. Further, the information collection and storage step S150 may store and share the secured, added, and analyzed pet information in a pet genome matchmaker database unit 10 connected to a publicly available social network.

The pet matching recommendation step S160 may receive a matching request from a matching applicant, receive at least one of genetic condition information, non-genetic condition information, external condition information, and introverted condition information about the desired matching target, and recommend a pet having the highest degree of matching with each condition information among the information stored in the information collection and storage step S150. In other words, it is possible to find and provide a pet that matches the conditions of the matching applicant in such a manner as to perform manual filtering according to the conditions or criteria desired by the matching applicant. As such, the pet matching recommendation step S160 may score the matching fitness between the conditions desired by the matching applicant, that is, genetic information, non-genetic information, external condition information, and introverted condition information, such as the type, breed, age, presence of disease, physical characteristics, and personality characteristics about the pet desired for matching, and the genetic information, non-genetic information, external condition information, and introverted condition information about each pet stored in the information collection and storage step S150, thereby providing information about the fittest pets.

Further, the pet matching recommendation step S160 may obtain analysis information through the gene analysis step S110 and the bioinformatics information analysis step S120 using the specimens collected from the pet of the matching applicant and recommend the pet with the highest degree of matching with the analysis information among the information stored in the information collection and storage step S150 or an appropriate number of pets in order from the highest matching degree. In other words, the pet matching recommendation unit 160 may automatically filter and provide a list of a few highest pets with a high degree of fitness based on various analysis information and input information.

Further, as the basis for judging the matching fitness, it is possible to search, filter, and provide information about the pet suitable for breeding with the matching applicant's pet through the process of scoring the matching fitness for the above-described epigenetic information (traits or characteristics obtained by environment and training) and bioinformatics information (information, such as genetic variation, physical characteristics, and characters that may appear during breeding).

Meanwhile, the pet matching recommendation step S160 may analyze, predict, and additionally provide at least one of genetic disease risk information about each pet based on the information stored in the information collection and storage step S150, and virtual appearance feature information, introverted feature information, health feature information (longevity potential), physical feature information (intelligence, body mass index, body hair thickness), and genetic disease risk information about the offspring from breeding with the recommended pet. In other words, it is possible to provide, in the form of a report, information about the potential genetic risk to the animal as well as the genetic risk to the offspring to be born by breeding with the recommended pet, through the genetic information about the matching applicant's pet.

Further, the pet matching recommendation step S160 may provide a visualization service for predicting the shape of the offspring based on the genetic information about the mating target. As shown in FIG. 3, it is possible to predict, visualize, and provide external information about the offspring of the second generation that are born when two first-generation cats virtually breed. More specifically, it is possible to provide a visualization service (providing photos or images) for the phenotype of the second generation that may appear by breeding, based on the shape of the ears, the color of the hair, and the shape of the nose. Such visual information may be provided by predicting the health, character, and physical characteristics of the offspring to be born at the time of breeding.

Meanwhile, the pet matching recommendation step S160 may also provide a visualization system for predicting the shape of the offspring based on the non-genetic information about the mating target. For example, the appearance of the offspring to be born by crossbreeding with a Chihuahua and a Jindo may be provided in a non-genetic photosynthesis scheme. If pet owners register two photos, the visualization service may predict and show an intermediate (mixed) face through a face synthesis algorithm based on the registered photo information.

The pet matching recommendation step S160 may calculate the degree of breeding fitness between pets according to heterogeneity of the major histocompatibility complex (MHC) gene and include and provide it in the virtual genetic disease risk information for the offspring to be born by breeding of the pets. Here, the major histocompatibility complex is a polymorphic gene, so it may have a very large number of alleles. Almost all humans as well as animals have different MHC alleles. There are two main types, type I and type II. Type I exists in almost all cells, and the T cell recognizes the MHC-1 molecule positioned on the surface of each cell to determine whether it is its own cell or not. If it turns out that it is not its own cell, the T cell destroys the cell. Because of this, when an organ transplant is received from another person, rejection occurs and the rejection must be suppressed for life through specific drugs.

As such, MHC genes perform the function of controlling the immune system to recognize and defend against foreign invaders, such as viruses or bacteria. It is possible to determine the risk of a genetic disease to the offspring by calculating the fitness of breeding according to the heterogeneity of the MHC gene and provide information thereabout. In other words, it is to predict the risk of genetic diseases of the offspring. It is possible to quantify the genetic risk inferred from the polymorphisms of genetic diseases and general diseases that occur in pets and humans. More specifically, it is possible to calculate and provide the predictive genetic risk used in human genetic testing and the heritable risk through mutation derived from a rare disease, separately and in combination.

Meanwhile, the pet matching recommendation step S160 according to the present embodiment may minimize the genetic disease according to the degree of kinship and the genetic disease probability score (carrier test), while maintaining the optimal pure blood breed.

Further, the pet matching recommendation step S160 may interwork with a system implemented in the form of an application or website that matches a pet breeding target online, so that the consumer (pet owner) may easily check the genome/gene analysis result through reports or various media.

Further, the pet matching recommendation step S160 may prevent genetic disease by examining the genetic information and non-genetic information based on the desired conditions for the fitness of breeding with a specific pet when breeding with the specific pet is desired and provide information about the risk of genetic diseases of current pets and the risk of future offspring (carrier test) according to the genotype of the future mating partner and may be used in various fields, such as breeding grounds, pet shops, and veterinary hospitals.

The genome-based matchmaking information providing method S100 for pets according to the present embodiment may provide owners with a function of securing and authenticating data for proof of pedigree relationship (authentication) of the pet that has been sold and provide a portal function through which the pet owner may easily manage information about his/her pet through the information collection and storage step S150 that is a pet genealogy DB. In other words, it is possible to manage the information even after the death of the pet.

Further, it is possible to judge the degree of kinship between pets through sample analysis. The range of the degree of difference in pet genetic variation may be previously defined for each species (e.g., distance between breeds of dogs, distance between breeds of cats), and the distance of animals falling in the range may be provided as the concept of degree of kinship of pets.

Meanwhile, the genome-based matchmaking information providing method S100 for pets according to the present embodiment is able to genetically prove and manage the genealogy (or pedigree) of animals with the most certainty and, If genomic information is stored when a pet dies, provide information related to the pet so that the animal closest to the pet may be adopted in the future.

Further, in the genome-based matchmaking information providing method S100 for pets, the genetic information may be stored in a storage medium, such as USB drive, to be easily identified online or on computer when the pet dies in the future.

Further, when expensive racehorses or stallions as well as pets are stolen or replaced, ownership may be proved through genetic testing and comparison with registered DB information. It may be a very reliable genetic test because it uses hundreds of thousands of markers (genotypes/biochips) that utilize a large amount of genetic information or sequences the entire genome of a pet.

While the present invention has been shown and described with reference to exemplary embodiments of the genome-based matchmaking social network system for pets and genome-based matchmaking information providing method for pets, it will be apparent to those of ordinary skill in the art that various changes in form and detail may be made thereto without departing from the spirit and scope of the disclosure as defined by the following claims.

## Claims

1. A pet genome-based matchmaking social network system, comprising:
a gene analyzer that provides gene analysis information through gene analysis using a specimen collected from a pet;
a bioinformatics information analyzer that provides at least one of genetic variation feature information, external feature information, and introverted feature information that appears upon breeding through bioinformatic analysis based on the gene analysis information;
a basic information input unit that receives user basic information about an owner of the pet and genetic feature information and non-genetic feature information about the pet from the owner of the pet;
a database unit that collects and stores information obtained and inputted through the gene analyzer, the bioinformatics information analyzer, and the basic information input unit; and
a pet matching recommendation unit that receives a matching request from a matching applicant, receives at least one condition information among genetic condition information, non-genetic condition information, external condition information, and introverted condition information about a desired matching target to recommend a pet with a highest degree of matching with the condition information among the information stored in the database unit or obtains analysis information through the genetic analyzer and the biogenetics information analyzer using a specimen collected from the matching applicant's pet to recommend a pet with a highest degree of matching with the analysis information among the information stored in the database unit.

2. The pet genome-based matchmaking social network system of claim 1, further comprising an epigenetic analyzer that provides growth and physiological expression feature information about the pet through epigenetic analysis using the specimen collected from the pet, wherein the database unit additionally collects and stores the growth and physiological expression feature information.

3. The pet genome-based matchmaking social network system of claim 2, wherein the growth and physiological expression feature information includes information according to a result of a structural change in chromatin due to DNA methylation or histone protein modification.

4. The pet genome-based matchmaking social network system of claim 2, wherein the pet matching recommendation unit analyzes, predicts, and additionally provides at least one of genetic disease risk information for each pet based on the information stored in the database unit and virtual external feature information, introverted feature information, health feature information, physical feature information, genetic disease risk information, and growth and physiological expression feature information for offspring resultant from breeding with a recommended pet.

5. The pet genome-based matchmaking social network system of claim 4, wherein the pet matching recommendation unit calculates a breeding fitness between pets according to a heterogeneity of a major histocompatibility complex (MHC) gene and includes and provides the breeding fitness in the virtual genetic disease risk information for the offspring resultant from breeding.

6. A pet genome-based matchmaking information providing method, comprising:
a gene analysis step that provides gene analysis information through gene analysis using a specimen collected from a pet, by a gene analyzer;
a bioinformatics information analysis step that provides at least one of genetic variation feature information, external feature information, and introverted feature information that appears upon breeding through bioinformatic analysis based on the gene analysis information, by a biogenetics information analyzer;
a basic information input step that receives user basic information about an owner of the pet and genetic feature information and non-genetic feature information about the pet from the owner of the pet, by a basic information input unit;
an information collection and storage step that collects and stores information obtained and inputted through the gene analyzer, the bioinformatics information analyzer, and the basic information input unit, by a database unit; and
a pet matching recommendation step that, by a pet matching recommendation unit, receives a matching request from a matching applicant, receives at least one condition information among genetic condition information, non-genetic condition information, external condition information, and introverted condition information about a desired matching target to recommend a pet with a highest degree of matching with the condition information among the information stored in the database unit or obtains analysis information through the genetic analyzer and the biogenetics information analyzer using a specimen collected from the matching applicant's pet to recommend a pet with a highest degree of matching with the analysis information among the information stored in the database unit.

7. The pet genome-based matchmaking information providing method of claim 6, further comprising an epigenetic analysis step that provides growth and physiological expression feature information about the pet through epigenetic analysis using the specimen collected from the pet, by an epigenetic analyzer, wherein the information collection and storage step additionally collects and stores the growth and physiological expression feature information.

8. The pet genome-based matchmaking information providing method of claim 7, wherein the growth and physiological expression feature information includes information according to a result of a structural change in chromatin due to DNA methylation or histone protein modification.

9. The pet genome-based matchmaking information providing method of claim 7, wherein the pet matching recommendation step analyzes, predicts, and additionally provides at least one of genetic disease risk information for each pet based on the information stored in the database unit and virtual external feature information, introverted feature information, health feature information, physical feature information, genetic disease risk information, and growth and physiological expression feature information for offspring resultant from breeding with a recommended pet.

10. The pet genome-based matchmaking information providing method of claim 9, wherein the pet matching recommendation step calculates a breeding fitness between pets according to a heterogeneity of a major histocompatibility complex (MHC) gene and includes and provides the breeding fitness in the virtual genetic disease risk information for the offspring resultant from breeding.
